# EUROPEAN PATENT APPLICATION

(11) **EP 3 444 013 A1**
(43) Date of publication of application: **20.02.2019**
(21) Application number: 17186420.0
(22) Date of filing: 16.08.2017
(51) Int. Cl.: A62B 18/00, A41D 13/11, A61B 5/087, A61M 16/00

(54) **A BREATHING CYCLE MONITORING DEVICE AND CONTROL METHOD**

(71) Applicant: Koninklijke Philips N.V., 5656 AE Eindhoven (NL)
(72) Inventor: SHI, Jun, 5656 AE Eindhoven (NL); ZHANG, Peng, 5656 AE Eindhoven (NL); SU, William, 5656 AE Eindhoven (NL); CHEN, Weizhong, 5656 AE Eindhoven (NL)
(74) Representative: de Haan, Poul Erik

(57) **Abstract**

The invention provides a breathing cycle monitoring device. A device is provided that incorporates a mask for covering the nose and/or mouth of a user, a first temperature sensor, a second temperature sensor and a controller. The mask has an air-permeable mask wall which defines a mask volume. In use, a microclimate exists inside the mask volume. The first temperature sensor is located inside the mask volume so that it is able to detect a first temperature of the air inhaled and exhaled inside the microclimate. The second temperature sensor is located outside the mask wall, at or adjacent to a surface of the air-permeable mask wall, so that it is able to detect a second temperature of the same air outside the microclimate. The controller compares the first and second temperatures across the air-permeable mask wall and is adapted to calculate a temperature difference parameter from the first and second temperatures. In doing so, the controller is able to determine a breathing cycle of the user.

## Description

### FIELD OF THE INVENTION

This invention relates to a breathing cycle monitoring device and control method particularly to a breathing cycle monitoring device containing temperature sensors.

### BACKGROUND OF THE INVENTION

Air pollution is a worldwide concern. The World Health Organization (WHO) estimates that 4 million people die from air pollution every year. Part of this problem is the outdoor air quality in cities. Nearly 300 smog-hit cities fail to meet national air quality standards.

Official outdoor air quality standards define particle matter concentration as mass concentration per unit volume (e.g. µg/m³). A particular concern is pollution with particles having a diameter less than 2.5 µm (termed "PM2.5") as they are able to penetrate into the gas exchange regions of the lung (alveoli), and very small particles (<100 nm) may pass through the lungs to affect other organs.

Since this problem will not improve significantly on a short time scale, a common way to deal with this problem is to wear a mask which provides cleaner air by filtration and the market for masks in China and elsewhere has seen a great surge in recent years. For example, it is estimated that by 2019, there will be 4.2 billion masks in China.

However, during use, the temperature and relative humidity inside the mask increases and combined with the pressure difference inside the mask relative to the outside, makes breathing uncomfortable. To improve comfort and effectiveness, a fan can be added to the mask which draws in air through a filter. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask.

Furthermore, in a conventional non-powered mask, inhalation also causes a slight negative pressure within the mask which leads to leakage of the contaminants into the mask, which leakage could prove dangerous if these are toxic substances. A powered mask delivers a steady stream of air to the face and may for example provide a slight positive pressure, which may be determined by the resistance of an exhale valve, to ensure that any leakage is outward rather than inward.

There have been numerous approaches to improving the user experience when wearing a powered mask. The approaches have tended to focus on regulation of fan speeds, both to improve user comfort and to improve the electrical efficiency of the fan.

For example, GB 2 032 284 discloses a respirator in which the pressure inside a mask is measured by a pressure sensor and the fan speed is varied in dependence on the sensor measurements.

Differential pressure sensors which determine a difference in pressure between air outside the device and air inside the device are thus often used in powered masks. Differential pressure sensors provide accurate monitoring of the breathing cycle of the user because there is a minimal time lag between the detected variation of differential pressure over time and the inhalation and exhalation timing of the user. This time lag is of the order of milliseconds or thousandths of a second. However, differential pressure sensors are expensive.

Temperature sensors are a cheaper alternative but there is a time lag when detecting the temperature such that the detected temperature is not a true reflection of the real-time temperature. This time lag is of the order of seconds such as 2-8 seconds, which is significant when compared to the duration of the breathing cycle (typically 3-5 seconds for a healthy adult at rest). This time lag also varies from person to person. Therefore, when using a temperature sensor to regulate the fan speed in masks, the time lag may cause the fans to act in opposition to the breathing cycle of the user. For example, an inlet fan may be turned on when the user is exhaling. This makes breathing in masks uncomfortable.

Therefore, there exists a need for cheaper alternative sensors for detecting the breathing cycle of a user in masks with a reduced time lag between the detected variation of the parameter over time and the inhalation and exhalation timing of the user.

### SUMMARY OF THE INVENTION

The invention is defined by the claims.

According to examples in accordance with an aspect of the invention, there is provided a breathing cycle monitoring device comprising:
a mask for covering the nose and/or mouth of a user, the mask having an air-permeable mask wall which defines a mask volume;
a first temperature sensor adapted to detect a first temperature, inside the mask volume;
a second temperature sensor adapted to detect a second temperature, at or adjacent to a surface of the air-permeable mask wall outside the mask volume; and
a controller which is adapted to:
   calculate a temperature difference parameter from the first and second temperatures; and
   determine a breathing cycle of the user based on the calculated temperature difference parameter.

There is proposed a concept of using two temperature sensors to detect a temperature change across a mask wall of air inhaled and exhaled by a user. In this way, the temperature change across the mask wall is monitored rather than only the temperature inside the mask. Therefore, the invention uses two (or more) temperature sensors instead of one to more quickly determine the breathing cycle of a user.

In use, a microclimate exists inside the mask. A temperature sensor detects the temperature of air inhaled and exhaled by the user inside the microclimate and another temperature sensor detects the temperature of the same air outside the microclimate before or after it has passed across the mask wall (but not all the way to the ambient surroundings). Generally, the air inside the mask is hotter than the air outside the mask because of the existence of the microclimate and the fact that ventilation inside the mask is poorer than outside. Therefore, the temperature inside the mask tends to be higher than the temperature outside the mask. This creates a temperature difference across the mask wall.

The controller processes the temperatures across the mask wall and is adapted to calculate a temperature difference parameter. This temperature difference parameter provides more accurate information regarding the breathing cycle of a user. Thus, the sensing delay of a temperature sensor, which may be 2-8 seconds, becomes irrelevant when using two temperature sensors either side of a mask wall to monitor the breathing cycle of a user.

By using two temperature sensors to detect a temperature change across the mask wall of air inhaled and exhaled by the user, the breathing cycle of a user is accurately determined. This has the benefit that cheap temperature sensors can replace expensive differential pressure sensors to accurately determine the breathing cycle of a user. Thus, the device of the invention uses inexpensive components and the device has wide applicability. The sensors may perform the dual role of inhalation/exhalation detection as well as providing feedback information for use in controlling the comfort of the mask.

By determining the breathing cycle of a user, the device may be used to monitor health in a range of situations such as walking, running etc. Alternatively, the device may control additional components in response to the determined breathing cycle, such as a fan arrangement in a powered mask.

It will therefore be appreciated that the inventors have realized that two temperature sensors can be used instead of differential pressure sensors to accurately determine the breathing cycle of a user and that the device can be used in a range of scenarios.

The second temperature sensor may for example be open to only one side, such that for example one side is sealed with heat insulation material (insulation side) and the other side is provided the heat contact side (sensing side). The sensing side is toward the mask wall and the insulation side is toward the outside. In this way, the second temperature sensor is adapted to detect the temperature adjacent to a surface of the air-permeable mask wall, outside the mask volume rather than the ambient temperature.

In one example, the air-permeable mask wall is a filter. In this way, the second temperature sensor detects the second temperature through the filter of the mask. The purpose of a mask is to filter and provide clean air to a user. The filter may form all or just a part of the air-permeable mask wall.

The temperature difference parameter may be calculated based on the first and second temperatures. Preferably, the temperature difference parameter is calculated by subtracting the first temperature from the second temperature. This is a simple calculation to accurately determine the breathing cycle of the user.

In another aspect, the second temperature sensor is mounted on the outside surface of the air-permeable mask wall. This ensures that the second temperature sensor remains in close proximity to the air-permeable mask wall.

Preferably, the second temperature sensor faces the outside surface of the air-permeable mask wall. This ensures that the second temperature sensor detects the temperature of air inhaled and exhaled by the user before or after it has passed across the mask wall and not the temperature of ambient air.

The device may be further adapted to transmit the data relating to the breathing cycle to an external device. This would enable health monitoring.

The device may further comprise:
a fan arrangement for ventilating the mask, wherein the controller is further adapted to:
   control the fan arrangement in synchronism with the breathing cycle of the user.

In this way, the breathing cycle of the user is fully assisted by the fan arrangement of the mask, making breathing in the mask more comfortable.

The fan arrangement may comprise an inlet fan and an outlet fan. This would improve ventilation of the mask to reduce the temperature and relative humidity inside the mask. An inlet fan brings fresh air into the mask and an outlet fan expels the air breathed out by the user from inside the mask to the outside.

The controller may be adapted to operate the inlet fan at a first speed during inhalation and a second, lower, speed during exhalation, and operate the outlet fan at a third speed during exhalation and a fourth, lower, speed during inhalation. In this way, the breathing cycle is fully assisted. The inlet and outlet fans synchronize with the breathing cycle of the user; the inhalation cycle is assisted by the inlet fan and the exhalation cycle is assisted by the outlet fan.

The second speed and the fourth speed may be zero. This minimizes battery use when the breathing cycle is in the opposite phase to the respective fan.

In another aspect of the invention, there is provided a method of controlling a breathing cycle monitoring device which device comprises: a mask for covering the nose and/or mouth of a user, the mask having an air-permeable mask wall which defines a mask volume; a first temperature sensor inside the mask volume; and a second temperature sensor at or adjacent to a surface of the air-permeable mask wall outside the mask volume, wherein the method comprises:
detecting a first temperature using the first temperature sensor;
detecting a second temperature using the second temperature sensor;
calculating a temperature difference parameter from the first and second temperatures; and
determining a breathing cycle of the user based on the calculated temperature difference parameter.

The device may further comprise a fan arrangement and thus the method may further comprise:
ventilating the mask using the fan arrangement; and
controlling the fan arrangement in synchronism with the breathing cycle of the user.

In another aspect of the invention, there is provided a computer program comprising computer program code means which is adapted, when said computer program is run on a computer, to implement the method defined above.

### BRIEF DESCRIPTION OF THE DRAWINGS

Examples of the invention will now be described in detail with reference to the accompanying drawings, in which:
Fig. 1 shows the differential pressure versus time detected using a differential pressure sensor and the temperature versus time detected using a temperature sensor during breathing cycles;
Fig. 2 shows a mask containing a first temperature sensor and a second temperature sensor;
Fig. 3 shows the temperature versus time, firstly determined using a temperature sensor inside the mask and secondly determined using a temperature sensor adjacent to the outside the mask;
Fig. 4 shows one example of the components of a mask containing a first temperature sensor, a second temperature sensor, an inlet fan and an outlet fan;
Fig. 5 shows the differential pressure versus time and the temperature difference parameter versus time during breathing cycles; and
Fig. 6 shows a device operating method of the invention.

### DETAILED DESCRIPTION OF THE EMBODIMENTS

The invention provides a breathing cycle monitoring device. A device is provided that incorporates a mask for covering the nose and/or mouth of a user, a first temperature sensor, a second temperature sensor and a controller. The mask has an air-permeable mask wall which defines a mask volume. In use, a microclimate exists inside the mask volume. The first temperature sensor is located inside the mask volume so that it is able to detect a first temperature of the air inhaled and exhaled inside the microclimate. The second temperature sensor is located outside the mask wall, at or adjacent to a surface of the air-permeable mask wall, so that it is able to detect a second temperature of the same air outside the microclimate. The controller compares the first and second temperatures across the air-permeable mask wall and is adapted to calculate a temperature difference parameter from the first and second temperatures. In doing so, the controller is able to determine a breathing cycle of the user.

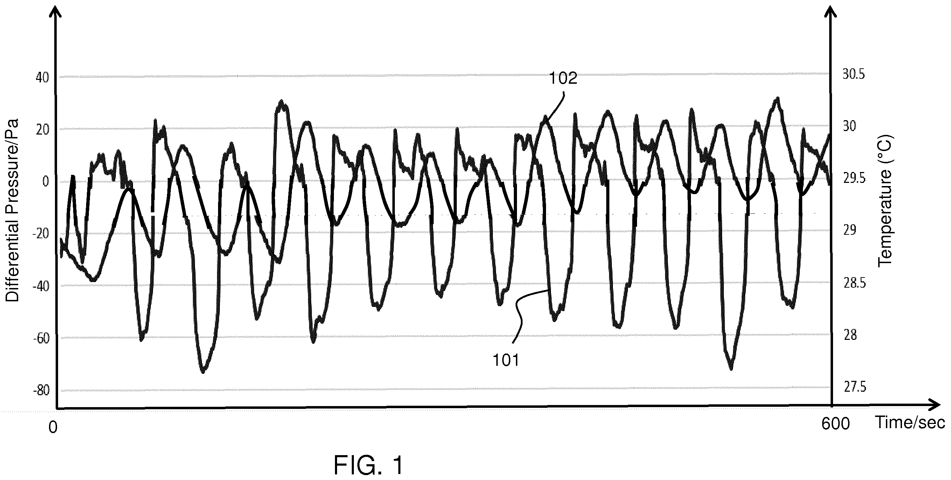

Fig. 1 shows the differential pressure versus time plot 101 detected using a differential pressure sensor and the temperature versus time plot 102 detected using a temperature sensor during breathing cycles. The x-axis is time/seconds. The left-hand y-axis is the differential pressure/Pa and the right-hand y-axis is the temperature/°C.

The breathing cycle is the pattern of inhalation and exhalation timings. There are a series of peaks and troughs in the breathing cycle corresponding to exhalation and inhalation, respectively. The breathing cycle may be represented by any suitable parameter which varies with inhalation/exhalation over time. Usually the breathing cycle is detected by monitoring differential pressure versus time, but other parameters may be used, such as temperature, relative humidity, oxygen and/or carbon dioxide concentration etc.

During inhalation, the differential pressure inside the mask decreases (negative pressure relative to the external ambient pressure) and the temperature inside the mask decreases. During exhalation, the differential pressure inside the mask increases (positive pressure) and the temperature inside the mask increases.

A differential pressure sensor provides accurate monitoring of the breathing cycle of the user because there is a minimal time lag in the detected variation of differential pressure over time, and hence the differential pressure closely follows the timing of inhalation and exhalation of the user. This time lag is of the order of milliseconds or thousandths of a second. Therefore, the differential pressure versus time plot depicted in Fig. 1 is an accurate representation of the breathing cycle of the user. However, differential pressure sensors are expensive.

In contrast, the use of a temperature sensor is cheaper. However, temperature sensors do not provide an accurate representation of the breathing cycle of the user because there is a time lag in detection of temperature over time, and hence a time lag with respect to the actual inhalation and exhalation timing of the user. This time lag is of the order of seconds such as 2-8 seconds for a temperature sensor, which is significant when compared to the duration of the breathing cycle (typically 3-5 seconds for a healthy adult at rest). This time lag also varies from person to person. Therefore, the temperature versus time plot depicted in Fig. 1 is an inaccurate representation of the breathing cycle of the user. The time lag associated with using a temperature sensor can be seen in Fig. 1 by comparing the temperature versus time plot with the differential pressure versus time plot. Therefore, when using a temperature sensor to regulate the fan speed in the case of masks, the time lag may cause the fans to act in opposition to the breathing cycle of the user. For example, an inlet fan may be turned on when the user is exhaling. This makes breathing in masks uncomfortable.

The applicant has found that a temperature change across the mask of air inhaled and exhaled by a user of the device allows the breathing cycle of the user to be more accurately determined than monitoring only the temperature of air inhaled and exhaled inside the mask.

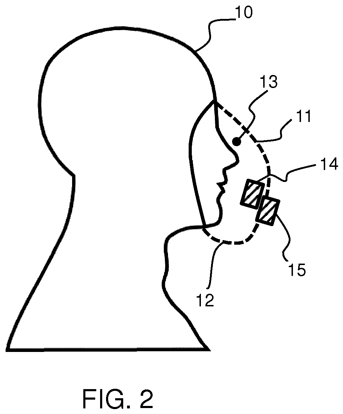

Fig. 2 shows a mask 11 of the invention containing a first temperature sensor 14 and a second temperature sensor 15.

The mask may additionally contain a fan arrangement for ventilating the mask. For example, the fan arrangement may comprise an inlet and/or an outlet fan.

A user 10 is shown wearing the face mask 11 which covers at least the nose and mouth of the user. The mask has an air-permeable mask wall 12 which defines a mask volume 13. The purpose of the mask is to filter air before it is breathed in by the user. For this purpose, in Fig. 2, the mask body itself acts as an air filter.

Alternatively, a filter may be provided only at the location of an inlet where a fan is located, in combination with an air-permeable mask wall which does not need to be a filter. This is possible if there is always a flow through the mask wall from inside the mask volume to outside the mask volume, for example because of the flow conditions set by the fan. The inlet fan may be positioned before or after the filter. In this arrangement, the exhaust air would not need to pass through the filter.

In all cases, the invention is based on temperature monitoring on both sides of an air-permeable layer, which may be a filter portion or may be an air-permeable layer with filtering performed separately.

The preferred arrangement of a mask in which the mask body is air-permeable and performs the filter function will be described.

Air is drawn into the mask volume 13 by inhalation. For an unpowered mask, air is drawn into the mask volume 13 from the outside across the air-permeable mask wall 12. However, if the mask additionally contains a fan arrangement, air may be drawn into the mask volume 13 from the outside via the fan arrangement, optionally in combination with the air-permeable mask wall 12 depending on the location and rotation speed of the fan arrangement. The temperature of the air surrounding the user's nose and/or mouth decreases during inhalation.

Air is drawn out of the mask volume 13 by exhalation. For an unpowered mask, air is drawn out of the mask volume 13 to the outside across the air-permeable mask wall 12. However, if the mask additionally contains a fan arrangement, air may be drawn out of the mask volume 13 to the outside via the fan arrangement, optionally in combination with the air-permeable mask wall 12 depending on the location and rotation speed of the fan arrangement. The temperature of the air surrounding the user's nose and/or mouth increases during exhalation.

The first temperature sensor 14 is adapted to detect a first temperature, inside the mask volume 13. Preferably, the first temperature sensor 14 is mounted inside the mask volume 13 adjacent to the mouth and/or nose of the user 10. The first temperature sensor 14 detects the temperature of the air inhaled and exhaled by the user. The first temperature fluctuates in response to the breathing cycle of the user.

The second temperature sensor 15 is adapted to detect a second temperature, at or adjacent to a surface of the air-permeable mask wall 12 outside the mask volume 13. The second temperature sensor 15 detects the temperature of the same air inhaled and exhaled by the user that was detected by the first temperature sensor 15. The second temperature sensor 15 does not just detect the temperature of ambient air; the second temperature fluctuates in response to the breathing cycle of the user and does not remain constant.

The second temperature sensor 15 is preferably mounted on the outside surface of the air-permeable wall 12. This ensures that the second temperature sensor 15 remains in close proximity to the air-permeable mask wall 12 so that the second temperature sensor 15 detects the air inhaled and exhaled by the user across the air-permeable mask wall 12. The second temperature sensor 15 may have a sensing side and a non-sensing side. For example, the temperature sensor component may be semi-closed, i.e. a having side sealed with a heat insulation material (an insulated side) and another side for heat contact (a sensing side). Therefore, the second temperature sensor preferably faces the outside surface of the air-permeable wall 12 and is insulated from the ambient surroundings. For example, the sensing side is towards the mask body and the insulated side is towards the outside/ambient air. This ensures that the second temperatures sensor 15 detects the temperature of air inhaled and exhaled by the user and not the temperature of ambient air.

The first temperature sensor may be the same or different to the second temperature sensor. However, as the difference between the first and second temperatures is typically less than 10 °C, the first and second temperature sensors are preferably the same. A suitable first and second temperature sensor is a Sensirion (Trade Mark) STS3x sensor.

In use, a microclimate exists in the mask volume 13. The first temperature sensor 14 detects the temperature of air inhaled and exhaled by the user inside the microclimate and the second temperature sensor 15 detects the temperature of the same air outside the microclimate. Generally, the air inside the mask volume 13 is hotter than the air outside the mask volume because of the existence of the microclimate and the fact that ventilation inside the mask volume 13 is poorer than outside. Therefore, the first temperature tends to be higher than the second temperature.

For an unpowered mask, the flow of inhaled air across the air-permeable mask wall 12 will be in the opposite direction to the flow of exhaled air across the air-permeable mask wall 12. Thus, a temperature change across the air-permeable mask wall 12 of air inhaled and exhaled by a user is detectable.

For a powered mask, the flow of inhaled air across the air-permeable mask wall 12 may be in the opposite direction to the flow of exhaled air across the air-permeable mask wall 12. For example, an inlet fan may assist inhalation and/or an outlet fan may assist exhalation. Alternatively, the flow of inhaled air across the air-permeable mask wall 12 may be in the same direction to the flow of exhaled air across the air-permeable mask wall 12 (i.e. always from inside the mask volume to outside the mask volume), depending on the position and rotation speed of the fan arrangement. For example, an inlet fan may be continuously operating such that air is always passing from the mask volume 13 to the outside across the air-permeable mask wall 12. Preferably, more air is removed from the mask volume 13 than exhaled so that additional air is supplied to the face. This increases comfort due to lowering relative humidity and cooling. In this case, although the flow of inhaled and exhaled air across the air-permeable mask wall 12 would be in the same direction, the flow rate of the air passing across the air-permeable mask wall 12 would be different during inhalation and exhalation so that a temperature change across the air-permeable mask wall 12 of air inhaled and exhaled by a user is detectable.

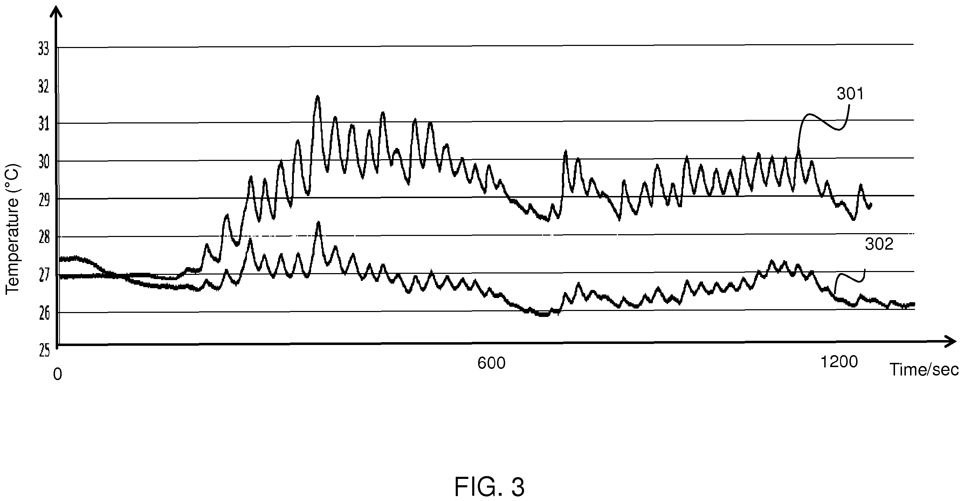

Fig. 3 shows the temperature versus time, firstly determined using a temperature sensor inside the mask and secondly determined using a temperature sensor adjacent to the outside the mask.

The first temperature is depicted as plot 301 and the second temperature is depicted as plot 302 in Fig. 3. Plots 301 and 302 diverge in temperature as the user starts inhaling and exhaling and the microclimate is established. Plot 301 detected inside the mask volume 13 is hotter than plot 302 detected outside the mask volume 13; there is a temperature drop between the inhaled and exhaled air inside the mask volume 13 and the same air outside the mask volume 13. The existence of a temperature difference on each side of the air-permeable mask wall 12 thus signifies that the device is in use and the first and second temperature sensors are detecting inhalation and exhalation.

As can be seen in Fig. 3, the peaks and troughs of plots 301 and 302 coincide and there is no delay between the detected first and second temperatures. However, the amplitude of the peaks and troughs are smaller for plot 302 than for plot 301. Plot 302 is effectively a trace of plot 301 before or after the inhaled and exhaled air of the user has passed through the air-permeable mask wall 12.

By detecting equal temperature on each side of the air-permeable mask wall 12, it can then be determined that the mask volume is not closed but is connected to ambient temperature on both sides. In this way, no inhalation and exhalation is detected. This can also signal that the device is not in use, and can thus be used to switch off the device to save power.

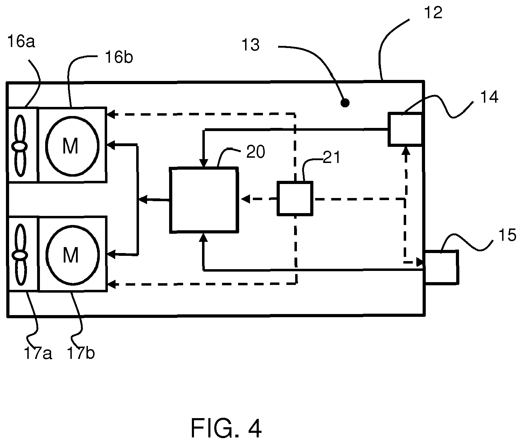

Fig. 4 shows one example of the components of a mask containing a first temperature sensor, a second temperature sensor, an inlet fan and an outlet fan. The same components as in Fig. 2 are given the same reference numbers.

In addition to the components shown in Fig. 2, Fig. 4 shows a controller 20, a local battery 21, an inlet fan 16 with an inlet fan blade 16a and an inlet fan motor 16b, and an outlet fan 17 with an outlet fan blade 17a and an outlet fan motor 17b.

The first and second temperatures are transmitted to the controller 20. The controller then calculates a temperature difference parameter from the first and second temperatures. In this way, the temperature change across the mask wall 12 of air inhaled and exhaled by a user is monitored rather than only the temperature of air inhaled and exhaled by the user inside the mask.

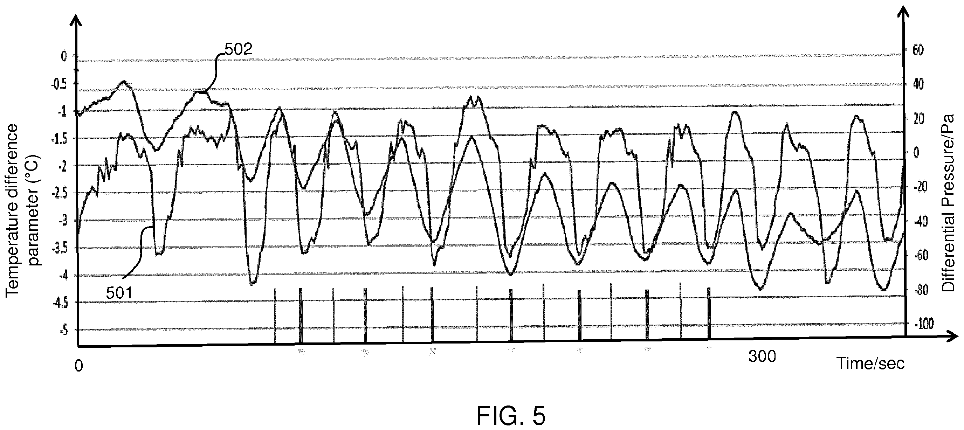

For example, the temperature difference parameter may be calculated by subtracting the first temperature from the second temperature (T2 - T1, where T2 is the second temperature and T1 is the first temperature). The results of this calculation can be seen in Fig. 5.

Fig. 5 shows the differential pressure versus time plot 501 and the temperature difference parameter versus time plot 502 during breathing cycles.

The temperature difference parameter versus time plot 502 is effectively a differential temperature versus time plot because the controller calculates the temperature difference across the air-permeable mask wall. It can be seen that the temperature difference parameter versus time plot 502 matches the differential pressure versus time plot 501 much more closely than the temperature versus time plot 102 matches the differential pressure versus time plot 101 in Fig. 1. The peaks and troughs of plots 501 and 502 match very well, as can be seen by the vertical markers along the bottom of Fig. 5 which align with the peaks and troughs of the two plots. Therefore, the controller is able to accurately determine a breathing cycle of the user based on the calculated temperature difference parameter. Therefore, the invention uses two temperature sensors to more accurately determine the breathing cycle of a user than one temperature sensor.

Considering the temperature difference parameter calculation in more detail, one example of a suitable calculation is the subtraction of the first temperature from the second temperature (T2 - T1). As the first temperature is higher than the second temperature, the resultant differential temperature is a negative value as seen in Figure 5. If this calculation is applied to the data of Fig. 3, the differential temperature value is the most negative when calculated for the peaks of the first and second temperatures. The differential temperature value is the least negative when calculated for the troughs of the first and second temperatures.

Calculating the differential temperature in this way transforms the temperature versus time plots into the mirror image plot (i.e. with approximately 180 degree phase shift with respect to the temperature plot). This mirror image plot such as plot 502 in Fig. 5 more closely matches the differential pressure versus time plot such as breathing cycle 501 in Fig. 5. Thus, the differential temperature versus time plot, like the differential pressure versus time plot, is now an accurate representation of the breathing cycle of the user.

It will of course be appreciated that alternative temperature difference parameter calculations can be performed on the first and second temperatures to determine a breathing cycle of the user. The timing of the peaks and troughs is of primary importance rather than the amplitudes of the signal. The temperature difference parameter may be f course calculated by subtracting the second temperature from the first temperature (T1 - T2) simply giving a sign-inverted version. The peaks of this plot would correspond to inhalation and the troughs of this plot would correspond to exhalation.

Alternatively, the first and second temperatures may be scaled by an appropriate factor before a subtraction is carried out. Thus, the temperature difference parameter may not be a simple direct subtraction. Indeed, the two temperature values may be combined using more complicated algebraic functions, but still with a link to the temperature difference, and hence a relationship with the temperature drop across the air-permeable membrane. Again, any such calculation may be used which is found to allow an accurate determination of the breathing cycle of a user.

The breathing cycle information may be implemented in a range of applications. For example, the controller may be adapted to transmit the data relating to the breathing cycle to an external device. For example, the external device may be a smartphone of the user and the transmission may be wireless via Wi-Fi, Bluetooth, ZigBee or other wireless technologies. Such a transmission would enable health monitoring by the user or another party.

The breathing cycle information may also be used to control fan speed in a powered mask. Fans generate a flow of air through the mask to reduce the temperature and relative humidity inside the mask and to regulate the pressure difference inside the mask relative to the outside. The fans are able to track the breathing cycle of the user, to make breathing in the mask more comfortable. For example, an inlet fan present in a mask may rotate during inhalation and an outlet fan may rotate during exhalation.

The device may further comprise a fan arrangement for ventilating the mask. The fan arrangement may comprise an inlet fan or an outlet fan to ventilate the mask. An inlet fan or an outlet fan assists the breathing of the user. For example, if an inlet fan is present, it may be switched on during inhalation and switched off during exhalation. Alternatively, if an outlet fan is present, it may be switched off during inhalation and switched on during exhalation.

When the mask is not in use, it may be switched off. In one design, the mask comprises a switch for starting and stopping the fan arrangement. This allows the user to have full control over when to start and stop the fan arrangement. For example, the user could ensure that the fan arrangement is switched off at all times when the mask is not in use. When the mask is switched on, the fan arrangement may start operating. Alternatively, there may instead be detection using a sensor of when the mask is being worn to provide automatic control of the fan arrangement. The mask may then go straight into its operating mode.

The fan arrangement may comprise an inlet fan 16 and an outlet fan 17 as depicted in Fig. 4. In this way, the inhalation/exhalation cycle is fully assisted. The inlet and outlet fans synchronize with the breathing cycle of the user; inhalation is assisted by the inlet fan to bring fresh air into the mask and exhalation is assisted by the outlet fan to expel the air breathed out by the user from inside the mask to the outside.

In one example, the fan motors 16b and 17b are electronically commutated brushless motors. Electronically commutated brushless motors are preferred for efficiency and longevity reasons.

In use, the inlet fan 16 and the outlet fan 17 may be run such that the controller 20 is adapted to operate the inlet fan 16 at a first speed during inhalation and a second, lower, speed during exhalation, and operate the outlet fan 17 at a third speed during exhalation and a fourth, lower, speed during inhalation. The first and second speeds of the inlet fan 16 and the third and fourth speeds of the outlet fan 17 refer to rotation speeds.

When a transition from exhalation to inhalation is determined, the controller 20 sends a signal to the inlet fan motor 16b to increase the rotation speed of the inlet fan blade 16a from the second speed to the first speed. The controller 20 also sends a signal to the outlet fan motor 17b to decrease the rotation speed of the outlet fan blade 17a from the third speed to the fourth speed. In this way, during inhalation, the inlet fan 16 is run at the first speed and the outlet fan 17 is run at the fourth speed. This compensates for the decrease in pressure inside the mask during inhalation.

Conversely, if a transition from inhalation to exhalation is determined, the controller 20 sends a signal to the outlet fan motor 17b to increase the rotation speed of the outlet fan blade 17a from the fourth speed to the third speed. The controller 20 also sends a signal to the inlet fan motor 16b to decrease the rotation speed of the inlet fan blade 16a from the first speed to the second speed. In this way, during exhalation, the outlet fan 17 is run at the third speed and the inlet fan 16 is run at the second speed. This compensates for the increase in pressure inside the mask during exhalation.

When both an inlet fan 16 and an outlet fan 17 are present, the second speed of the inlet fan 16 is preferably the same as the fourth speed of the outlet fan 17. This provides a consistent user experience, in terms of feel and sound.

The first speed of the inlet fan 16 may be the same as or different to the third speed of the outlet fan 17, depending on the design of the inlet and outlet flow paths of the mask and the differential pressure inside the mask created by the inlet fan 16 and the outlet fan 17. For example, if air is drawn into the mask through the filter and drawn out of the mask through a valve, the inlet fan 16 would need to generate a higher pressure than the outlet fan 17. This could be achieved using a first speed for the inlet fan 16 that is higher than the third speed for the outlet fan 17.

The second speed and the fourth speed may be zero or a minimum non-zero speed. Switching off the fans minimizes battery use when the breathing cycle is in the phase for which the respective fan is not needed. Alternatively, the second speed and the fourth speed could be non-zero. One of the benefits of running the inlet fan 16 at a minimum non-zero second speed and the outlet fan 17 at a minimum non-zero fourth speed is that the fans are run at a low idling speed which uses minimal power, but reduces latency. Further, continuously running the inlet and outlet fans at least at a minimum level ensures that there is minimal delay when switching the operation of the inlet fan to the outlet fan during the transition between inhalation and exhalation, and when switching the operation of the outlet fan to the inlet fan during the transition between exhalation and inhalation. Thus, the air flow in the mask may be synchronized more easily with the breathing cycle of the user, ultimately making breathing in the mask more comfortable.

The fan speeds may be tailored to the breathing of the user (e.g. breath frequency and tidal volume) and may be adjusted to take into account different breathing scenarios (e.g. exertion like walking and running).

The speeds to be used may be determined during a calibration process or they may be provided by the fan manufacturer. The calibration process for example involves analyzing the fan speed information over a period during which the user is instructed to inhale and exhale regularly with normal breathing. The captured fan speed information can then be used to determine the appropriate fan speeds. The controller may also provide for settings for the user to regulate the higher first and third speeds, and the lower second and fourth speeds, and any intermediate speeds.

In a most simple example, the rotation speeds of the inlet fan 16 and the outlet fan 17 alternate between two set values, with the changes in rotation speed implemented at the detected transitions between inhalation and exhalation.

There may also be a number of intermediate rotation speeds at which the inlet and outlet fans may be run between the first and third speeds and between the second and fourth speeds. However, the second and fourth speeds normally set the minimum rotation speed. The minimum rotation speed ideally provides an optimum balance between lag time and power efficiency. The first and third speeds are typically dependent on the breathing of the user (e.g. breath frequency and tidal volume) and could be adjusted to take into account different breathing scenarios (e.g. exertion like walking and running). In one simple embodiment, the first and third speeds set the maximum rotation speed. In this way, the first and third speeds ideally provide an optimum balance between lag time and power efficiency on the one hand, and assistance given to the user on the other.

The rotation speeds of the inlet and outlet fans are for example controlled by a pulse width modulation signal, whereby the duty cycle controls the rotation speed.

The mask may further comprise a valve. An inlet valve may be provided adjacent to the inlet fan, when present, and an outlet valve may be provided adjacent to the outlet fan, when present. Thus, the fan arrangement may comprise a fan and a valve. For example, the fan arrangement may comprise an inlet fan and an inlet valve, an inlet fan and an outlet valve, or an outlet fan and an outlet valve, or any combination of fans and valves. In one aspect, the mask further comprises a valve for exhausting air from inside the mask volume 13 to the outside. During inhalation, an outlet valve such as a check valve is closed due to the low pressure in the mask volume 13. When the user breathes out, air is exhausted through the outlet valve. This valve is opened to enable easy exhalation, but is closed during inhalation.

During inhalation, by closing the valve, it is prevented that unfiltered air is drawn in. The timing of the outlet valve is thus dependent on the breathing cycle of the subject. The outlet valve may be a simple passive check valve operated by the pressure difference across the air-permeable mask wall. However, it may instead be an electronically controlled valve.

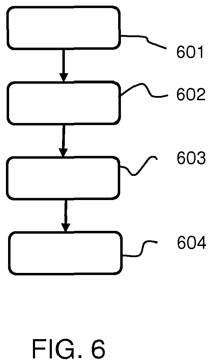

Fig. 6 shows a device operating method of the invention. The method is for controlling a device, which device comprises: a mask 11 for covering the nose and/or mouth of a user 10, the mask having an air-permeable mask wall 12 which defines a mask volume 13; a first temperature sensor 14 inside the mask volume; and a second temperature sensor 15 at or adjacent to a surface of the air-permeable mask wall outside the mask volume. The method comprises the following steps.

In step 601, a first temperature is detected using the first temperature sensor.

In step 602, a second temperature is detected using the second temperature sensor.

In step 603 a temperature difference parameter is calculated from the first and second temperatures.

In step 604, a breathing cycle of the user is determined based on the calculated temperature difference parameter.

The device may further comprise a fan arrangement and thus the method may further comprise ventilating the mask using the fan arrangement and controlling the fan arrangement in synchronism with the breathing cycle of the user.

The present invention also provides a computer program comprising computer program code means which is adapted, when said computer program is run on a computer, to implement the method of the invention.

The method of the present invention makes use of a controller, which can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g. one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Preferably, the mask further comprises a battery to power the first and second temperature sensors, the controller and the fan arrangement, when present.

The mask may be for covering only the nose and mouth (as shown in Fig. 2) or it may be a full face mask.

The example shown is a mask for filtering ambient air. However, the mask may be used with a breathing gas from an external supply, for example a breathing assistance device, such as a continuous positive air pressure (CPAP) system.

It will be seen that the invention may be applied to many different device designs such as respirators, with fan-assisted inhalation and exhalation, and with a mask volume formed by an air-permeable mask walk or a filter membrane.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

Any reference signs in the claims should not be construed as limiting the scope.

## Claims

1. A breathing cycle monitoring device comprising:
a mask (11) for covering the nose and/or mouth of a user (10), the mask having an air-permeable mask wall (12) which defines a mask volume (13);
a first temperature sensor (14) adapted to detect a first temperature, inside the mask volume (13);
a second temperature sensor (15) adapted to detect a second temperature, at or adjacent to a surface of the air-permeable mask wall (12) outside the mask volume (13); and
a controller (20) which is adapted to:
calculate a temperature difference parameter from the first and second temperatures; and
determine a breathing cycle of the user based on the calculated temperature difference parameter.

2. A breathing cycle monitoring device as claimed in claim 1, wherein second temperature comprises a first side with heat insulation material and a second, heat contact, side.

3. A breathing cycle monitoring device as claimed in claim 1 or 2, wherein the air-permeable mask wall (12) is a filter.

4. A breathing cycle monitoring device as claimed in any preceding claim, wherein the temperature difference parameter is calculated by subtracting the first temperature from the second temperature.

5. A breathing cycle monitoring device as claimed in any preceding claim, wherein the second temperature sensor (15) is mounted on the outside surface of the air-permeable mask wall (12).

6. A breathing cycle monitoring device, wherein the second temperature sensor (15) faces the outside surface of the air-permeable mask wall (12).

7. A breathing cycle monitoring device as claimed in any preceding claim, wherein the controller (20) is further adapted to transmit the data relating to the breathing cycle to an external device.

8. A breathing cycle monitoring device as claimed in any preceding claim, wherein the device further comprises:
a fan arrangement (16, 17) for ventilating the mask (11), wherein the controller (20) is further adapted to:
control the fan arrangement (16, 17) in synchronism with the breathing cycle of the user.

9. A breathing cycle monitoring device as claimed in claim 8, wherein the fan arrangement (16, 17) comprises an inlet fan (16) and an outlet fan (17).

10. A breathing cycle monitoring device as claimed in claim 9, wherein the controller (20) is adapted to operate the inlet fan (16) at a first speed during inhalation and a second, lower, speed during exhalation, and operate the outlet fan (17) at a third speed during exhalation and a fourth, lower, speed during inhalation.

11. A breathing cycle monitoring device as claimed in claim 10, wherein the second speed and the fourth speed are zero.

12. A method of controlling a breathing cycle monitoring device, which device comprises: a mask (11) for covering the nose and/or mouth of a user (10), the mask having an air-permeable mask wall (12) which defines a mask volume (13); a first temperature sensor (14) inside the mask volume (13); and a second temperature sensor (15) at or adjacent to a surface of the air-permeable mask wall (12) outside the mask volume (13), wherein the method comprises:
detecting a first temperature using the first temperature sensor (14);
detecting a second temperature using the second temperature sensor (15);
calculating a temperature difference parameter from the first and second temperatures; and
determining a breathing cycle of the user based on the calculated temperature difference parameter.

13. A method of controlling a breathing cycle monitoring device as claimed in claim 12, which device further comprises a fan arrangement (16, 17), wherein the method further comprises:
ventilating the mask (11) using the fan arrangement (16, 17); and
controlling the fan arrangement (16, 17) in synchronism with the breathing cycle of the user.

14. A computer program comprising computer program code means which is adapted, when said computer program is run on a computer, to implement the method of claims 12 or 13.
